# EUROPEAN PATENT APPLICATION

(11) **EP 0 880 954 A1**
(43) Date of publication of application: **02.12.1998**
(21) Application number: 97902659.8
(22) Date of filing: 12.02.1997
(51) Int. Cl.: A61F 13/15, C09J 7/00, B65D 65/40, D21H 19/32, D06M 15/65

(54) **ITEM-PACKAGING SHEET FOR SANITARY NAPKINS AND SANITARY NAPKINS PACKAGED IN SAID SHEET**

(30) Priority: 14.02.1996 JP 26475/96; 25.12.1996 JP 345361/96
(71) Applicant: NITTO DENKO CORPORATION, Osaka 567 (JP)
(72) Inventor: HIRAMATSU, Tsuyoshi, Ibaraki-shi, Osaka 567 (JP); TAKAHASHI, Makoto, Ibaraki-shi, Osaka 567 (JP); ITO, Takio, Ibaraki-shi, Osaka 567 (JP); NIINO, Takuya, Ibaraki-shi, Osaka 567 (JP)
(74) Representative: Grünecker, Kinkeldey, Stockmair & Schwanhäusser Anwaltssozietät
(86) International application number: JP9700363
(87) International publication number: WO9729723

(57) **Abstract**

This invention provides a packaging sheet for a sanitary napkin in which a release agent layer is formed on one side of a base material, wherein the release agent layer is one prepared by curing a release agent having an epoxy functional polyorganosiloxane as the main component by ultraviolet ray exposure, and the epoxy functional polyorganosiloxane contains a monovalent epoxy functional organic group in an amount of 1 to 20 mol % of the total organic groups, and a packaged article of the sanitary napkin in which the sanitary napkin having a pressure-sensitive adhesive layer formed on the surface is packaged in the packaging sheet.

The packaging sheet and packaged article of a sanitary napkin according to this invention do not cause tight release of the packaging sheen even if they are exposed to lights such as sunlight and fluorescent light until their use, so that the packaging sheet can be released from the napkin smoothly.

## Description

### TECHNICAL FIELD

This invention relates to a packaging sheet for use in individually packaging a sanitary napkin having provided thereon a pressure-sensitive adhesive to fix it to underwear, and to a packaged article in which a sanitary napkin having provided thereon a pressure-sensitive adhesive is packaged with the packaging sheet.

### BACKGROUND ART

Napkins in which a pressure-sensitive adhesive layer is partially formed on the surface to prevent displacement of sanitary napkins (hereinafter simply referred to as napkin) at the time of their use are already on the market.

Conventionally, napkins having a pressure-sensitive adhesive as described above were individually packaged, one by one in a plastic film etc. after protecting the pressure-sensitive adhesive layer with a release liner (separator). However, such a packaging mode has a problem in the generation of wastes such as the release liner at the use as well as the economical problem. In recent years, another mode in which napkins having a pressure-sensitive adhesive are packaged in a packaging material having a releasing function without using a release liner is becoming a mainstream.

As the packaging material (hereinafter referred to as "packaging sheet") which can directly package a napkin having a pressure-sensitive adhesive without using a release liner, various constructions have been proposed. For example, JP-A-3-184543 and JP-A-4-284237 (the term "JP-A" as used herein means an unexamined published Japanese patent application) disclose a packaging sheet as the packaging material for napkins having a pressure-sensitive adhesive, in which a silicone release agent layer, which can be cured by the irradiation of electron rays or ultraviolet rays, is formed on one side of a plastic film such as polyethylene.

However, in some of the cases of a packaging sheet having a release agent layer composed of silicone cured with ionizing radiation such as electron rays or ultraviolet rays, the following problem is caused. Namely, while napkin articles containing napkins packaged with the packaging sheets individually are displayed in the shop window or a consumer who purchased the napkin articles leaves them under a direct sunlight-exposed condition until their use, the exposure to direct sunlight causes the packaging sheet to gradually become a tight release state (that is, interface interaction between the release agent layer and the pressure-sensitive adhesive layer becomes strong), so that the packaging sheet becomes difficult to peel at the time of the use of napkins.

The present invention has been made by taking these problems into consideration, and the object is to provide a packaging sheet in which even if napkins packaged with the packaging sheet individually are exposed to sunlight, fluorescent light and the like until their use, the packaging sheet does not cause tight release and the packaging sheet can be released from the napkins smoothly when used, and a packaged article in which a sanitary napkin is packaged by the use of the packaging sheet.

### DISCLOSURE OF THE INVENTION

With the aim of resolving the aforementioned problems, the inventors of the present invention have conducted intensive studies and found as a result of the efforts that the problems can be resolved by preparing a packaging sheet by (1) using, as the release agent layer of the packaging sheet, a release agent which contains an epoxy functional polyorganosiloxane having a specified amount of monovalent functional organic groups as the main component and (2) curing the release agent by ultraviolet rays, and packaging a napkin with the packaging sheet, thereby achieving the present invention.

Accordingly, the present invention provides a packaging sheet for a sanitary napkin in which a release agent layer is formed on one side of a base material, wherein the release agent layer is one prepared by curing a release agent containing an epoxy functional polyorganosiloxane as the main component by ultraviolet ray irradiation, and the epoxy functional polyorganosiloxane contains a monovalent epoxy functional organic group in an amount of 1 to 20 mol % of the total organic groups (claims 1 and 2).

The present invention also relates to a packaged article in which a napkin, which has a pressure-sensitive adhesive layer formed on the surface to fix it to underwear, packaged with the packaging sheet (claim 3) and to a packaged article containing a napkin in which the pressure-sensitive adhesive layer formed on the surface of the napkin is comprised of a pressure-sensitive adhesive which contains a polymer having aliphatic double bonds between carbon atoms in the molecule as the main component (claim 4).

### BRIEF DESCRIPTION OF THE DRAWINGS

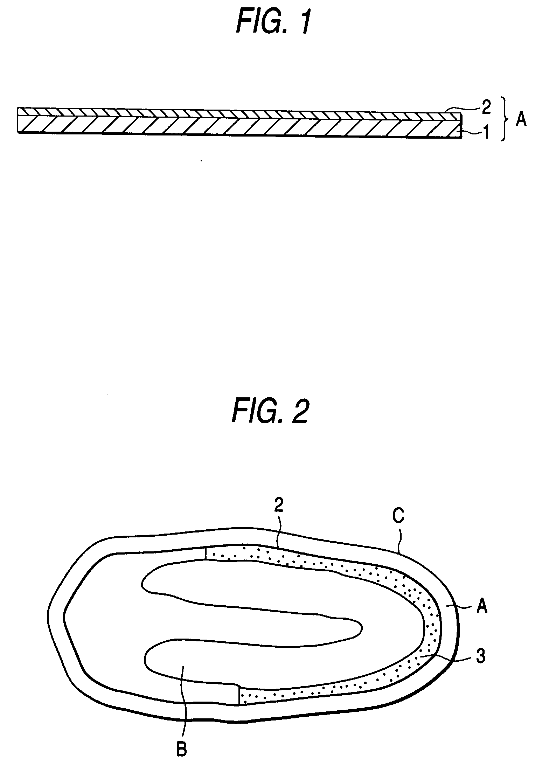

Fig. 1 is a sectional view showing the packaging sheet for individually packaging a sanitary napkin according to the present invention, and Fig. 2 is a schematic illustration showing the packaged article containing a sanitary napkin according to the present invention.

### BEST MODE OF CARRYING OUT THE INVENTION

The following describes the present invention with reference to the drawings.

Fig. 1 is a sectional view showing an illustrative example of the packaging sheet of the present invention, in which a release agent layer 2 is formed on one side of a base material 1 to constitute a packaging sheet A.

The epoxy functional polyorganosiloxane to be used as the main component of the release agent layer is one which can cause cationic curing reaction through ultraviolet ray irradiation in the presence of a cationic photo-curing catalyst (which will be described later) to thereby form a release agent layer.

In the present invention, it is necessary that monovalent epoxy functional organic groups occupy the proportion of from 1 to 20 mol %, preferably from 2 to 15 mol %, of the total organic groups (organic groups linked to polysiloxane chain as the main chain) of the epoxy functional polyorganosiloxane. If the amount of monovalent epoxy functional organic groups is less than 1 mol %, the curing rate becomes slow when ultraviolet rays are irradiated, thus causing considerable reduction of the productivity of the packaging sheet, and curing of the release agent also becomes insufficient, which may cause reduction of pressure-sensitive adhesive characteristics of the pressure-sensitive adhesive layer of the napkin which is in contact with the release agent layer.

On the other hand, if the amount of monovalent epoxy functional organic groups exceeds 20 mol %, when a napkin is packaged with a packaging sheet in which such an epoxy functional polyorganosiloxane is used as the release agent, generation of tight release becomes markedly frequent at the time of the exposure to lights such as sunlight.

According to the present invention, the epoxy functional polyorganosiloxane to be used as the release agent of the packaging sheet is not particularly limited, as long as it satisfies the aforementioned conditions, and can be selected arbitrarily from known epoxy functional polyorganosiloxane compounds. Examples of such epoxy functional polyorganosiloxane include those which have a structure represented by the following formula (1).

R¹ₘR²ₙSiO_{(4-m-n)/2} (1)

In the above formula, R¹ is a hydrogen atom or a monovalent hydrocarbon group, and examples of the monovalent hydrocarbon group include an alkyl group (e.g., methyl, ethyl, propyl, butyl, hexyl, octyl), an aryl group (e.g., phenyl, tolyl) and an alkenyl group (e.g., vinyl, allyl). Among these groups, a methyl group is desirable since good release property can be obtained.

R² is a hydrogen atom, a divalent hydrocarbon group, a monovalent hydrocarbon group or a monovalent epoxy functional organic group. The groups described in relation to R¹ can be exemplified as the monovalent hydrocarbon group. As the divalent hydrocarbon group, methylene, ethylene, propylene and the like can be exemplified. On the other hand, as the monovalent epoxy functional organic group, glycidyl, glycidoxy, 3,4-epoxycyclohexyl, 2,3-epoxycyclopentyl and the like can be exemplified.

In addition, m and n in the above formula each is an integer of from 0 to 3, and the total of m and n is an integer of from 0 to 3.

The epoxy functional polyorganosiloxane can be obtained, for example, by carrying out an addition reaction (hydrosilylation reaction) of an olefinic epoxy monomer such as 4-vinylcyclohexene oxide, allyl glycidyl ether, and 7-epoxy-1-octene with the base polymer, polymethylhydrogensiloxane, in the presence of a catalyst such as a platinum compound.

Alternatively, it may be obtained by carrying out partial cross-linking of the base polymer, polymethylhydrogensiloxane, to a polyorganosiloxane containing at least two alkenyl groups in one molecule and then effecting addition reaction with the olefinic epoxy monomers.

In addition, an epoxy functional polyorganosiloxane containing trifunctional siloxane units as described in JP-A-4-159322 can also be used.

According to the present invention, the cationic photo-curing catalyst which is added to the epoxy functional polyorganosiloxane to cure the release agent by ultraviolet ray irradiation is not particularly limited and any known compounds can be used, as long as it has compatibility with the epoxy functional polyorganosiloxane and is able to open the epoxy ring upon irradiation of ultraviolet ray.

Examples of such cationic photo-curing catalyst include onium salt compounds represented by formulae R³₂I⁺MXₙ⁻_{,} R³₃S⁺MXₙ⁻, R³₃Se⁺MXₙ⁻, R³₄P⁺MXₙ⁻ and R³₄N⁺MXₙ⁻. In these formulae, R³'s are the same or different and organic groups selected from the group consisting of
(1) a C₆ ∼ C₂₀ aromatic hydrocarbon group,
(2) a C₆ ∼ C₂₀ aromatic hydrocarbon group substituted with 1 to 4 monovalent organic groups or atoms selected from a C₁ to C₈ alkoxyl group, a C₁ to C₈ alkyl group, a nitrogen atom, a chlorine atom, a bromine atom, a cyano group, a carboxyl group and a mercapto group, and
(3) an aromatic heterocyclic group selected from a pyridyl group, a thiophenyl group and a pyranyl group.

Also, MXₙ⁻ is a non-basic and non-nucleophilic anion selected from the group consisting of BF₄⁻, PF₆⁻, AsF₆⁻, SbF₆⁻, SbCl₆⁻, HSO₄⁻, ClO₄⁻, B(C₆F₅)₄⁻ and the like.

According to the present invention, a diaryliodonium salt such as bis(dodecylphenyl)iodonium hexafluoroantimonate can be preferably used.

The addition amount of the cationic photo-curing catalyst is not particularly limited, but the amount is generally from 0.3 to 10 parts by weight, preferably from 0.5 to 6.0 parts by weight, based on 100 parts by weight of the epoxy functional polyorganosiloxane.

If the amount of the cationic photo-curing catalyst to be added is smaller than 0.3 part by weight, it will entail reduction of the reactivity (curing ability) of epoxy functional polyorganosiloxane, which is not desirable. Also, if the amount of the cationic photo-curing catalyst to be added is lager than 10 parts by weight, its effect to improve the reactivity (curing ability) becomes small which is not desirable from the economical point of view.

As occasion demands, compatibility of the epoxy functional polyorganosiloxane with the cationic photo-curing catalyst can be improved and release characteristics of the release agent layer can be controlled by adding known additive components to the release agent of the present invention which contains the epoxy functional polyorganosiloxane as the main component. Examples of such additive components include a polyether compound described in JP-A-4-126767, an alkylphenol having 8 to 16 carbon atoms and a phenolalkyl-substituted organodisiloxane described in JP-A-3-115425 and an α-methylstyrene compound and a p-substituted styrene compound described in JP-A-4-11678.

Kinds of the base material to be used in the packaging sheet are not particularly limited, and it can be arbitrarily selected from polyolefin plastic films such as of polystyrene, polypropylene, plastic films such as of polyester, nylon, cellophane, paper products such as wood-free paper, extended paper (Clupack), or glassine paper and a laminated base material formed by laminating a plastic film on non-woven fabric.

Among these materials, polyethylene film may be used most suitably because of its flexibility and good feel to the touch.

As occasion demands, the surface of the base material can be treated by corona discharge, embossing finish or print processing.

The packaging sheet of the present invention is prepared by coating a release agent containing the aforementioned epoxy functional polyorganosiloxane as the main component on the surface of the base material and then irradiating ultraviolet rays.

Coating of the release agent on the base material can be carried out by arbitrarily selecting known coating apparatus such as direct gravure coater, offset gravure coater, roll coater, bar coater, or die coater.

Coating mode of the release agent layer to be formed on the base material surface is not particularly limited; for example, the release agent may be coated on the entire area of one side of the base material or coated partially on only an area where it contacts with the pressure-sensitive adhesive layer arranged on a napkin.

Though not particularly limited, the amount of the release agent layer to be coated may be generally from 0.1 to 5 g/m², preferably from 0.1 to 2 g/m². If the coating amount is smaller than 0.1 g/m², uniform coating on the base material surface becomes difficult, thus easily generating coating irregularity (pin holes). Also, even if the coating amount exceeds 5 g/m², changes in the releasing ability are small, so that is undesirable economically.

After coating of the release agent on the base material surface, the irradiation of ultraviolet rays can be effected arbitrarily selecting and using a known ultraviolet ray lamp such as an electrode high pressure mercury lamp, metal halide lamp or an electrode-less microwave lamp.

Fig. 2 is a schematic illustration showing an example of the packaged article containing a napkin according to the present invention, in which a napkin B having a pressure-sensitive adhesive layer 3 is wrapped individually in the packaging sheet A of the present invention. In this case, a napkin is packaged with the packaging sheet A in such a manner that the sheet contacts with the pressure-sensitive adhesive layer of the napkin via the release agent layer 2.

Kinds of the napkin which is used in the packaged article are not particularly limited, and any one of the generally used napkins can be used.

Also, kinds of the pressure-sensitive adhesive layer to be formed on the surface of napkins are not particularly limited, and any known pressure-sensitive adhesives such as rubber based pressure-sensitive adhesives or acrylic pressure-sensitive adhesives can be used.

Particularly, the present invention can exert markedly significant effect (no generation of tight release in the packaging sheet even when the packaged article containing a napkin is exposed to sunlight and the like), when the pressure-sensitive adhesive layer formed on the surface of napkins is comprised of a pressure-sensitive adhesive which contains, as the main component, a polymer having aliphatic double bonds between carbon atoms in the molecule. It is guessed that such an effect is obtained by a mechanism in which, in the case of using a polymer as the main component of the pressure-sensitive adhesive contains aliphatic double bonds between carbon atoms in the molecule, when the pressure-sensitive adhesive is exposed to light such as sunlight, the aliphatic double bonds in the molecule are cleaved and generate radicals, and these radicals react with the release agent of the packaging sheet which is in contact with the pressure-sensitive adhesive layer, thereby strengthening mutual reactions between surfaces of the pressure-sensitive adhesive layer and release agent layer.

Examples of the pressure-sensitive adhesive having, as the main component, a polymer which contains aliphatic double bonds between carbon atoms in the molecule include a natural rubber pressure-sensitive adhesive which contains natural rubber as the main component, a synthetic rubber pressure-sensitive adhesive which contains a synthetic rubber such as styrene-butadiene rubber, isoprene rubber, or butyl rubber as the main component and a hot melt type pressure-sensitive adhesive which contains a block polymer such as styrene-isoprene block copolymer (SIS) or styrene-butadiene block copolymer (SBS) as the main component.

Also, examples of the polymer which contains aliphatic double bonds between carbon atoms in the molecule include a polymer produced by hydrogenating a block polymer such as styrene-isoprene block copolymer or styrene-butadiene block copolymer, as long as the polymer is not completely hydrogenated due to its production method so that aliphatic double bonds partially remain in the molecule. Examples of such a type of polymer include styrene-ethylene-butylene block copolymer (SEBS) in which all of the aliphatic double bonds in the molecule are not hydrogenated.

With regard to the pressure-sensitive adhesive layer to be formed on the surface of napkins, in addition to the pressure-sensitive adhesive having, as the main component, a polymer which contains aliphatic double bonds between carbon atoms in the molecule, an acrylic pressure-sensitive adhesive produced by cross-linking a polymer containing (meth)acrylic acid ester such as butyl (meth)acrylate or 2-ethylhexyl (meth)acrylate as the main component using a known cross-linking agent, can also be used.

In addition, as occasion demands, the pressure-sensitive adhesive may be blended with known additive agents such as a tackifier, an antioxidant, a pigment, and a filler.

### [EXAMPLES]

The following describes the present invention with reference to illustrative examples, but the invention is not restricted by these examples.

### (Inventive Example 1)

A release agent prepared by adding 1 part by weight of bis(dodecylphenyl)iodonium hexafluoroantimonate to 100 parts by weight of an epoxy functional polydimethylsiloxane to which 6 mol % of 4-vinylcyclohexene oxide based on the total methyl groups has been added (viscosity at 25°C, 350 cP) was coated on one side of a polyethylene film having a thickness of 25 µm and subjected to ultraviolet ray irradiation under the following coating and irradiation conditions, thereby preparing a packaging sheet of the present invention.

A hot melt type pressure-sensitive adhesive comprised of a styrene-isoprene block copolymer was coated in a thickness of 45 µm on one side of a polypropylene/polyethylene blend film (weight ratio: 6/4, 95 µm in thickness) to prepare a pressure-sensitive adhesive tape (50 mm in width, 150 mm in length), and the release agent layer of the packaging sheet described above was laminated on the pressure-sensitive adhesive layer of the thus prepared pressure-sensitive adhesive tape, thereby preparing a sample for use in the evaluation of peeling strength.

### Conditions for release agent coating

- Coating apparatus: offset gravure coater
- Coating amount: 1 g/m²
- Line speed: 20 m/min
- UV lamp: F-450 (H bulb) manufactured by FUSION
- Quantity of UV light: 120 W/cm

### (Inventive Example 2)

A sample for use in the evaluation of peeling strength was prepared in the same manner as described in Inventive Example 1, except that a mixture prepared by adding 1 part by weight of bis(dodecylphenyl)iodonium hexafluoroantimonate to 100 parts by weight of an epoxy functional polydimethylsiloxane to which 5 mol % of 4-vinylcyclohexene oxide based on the total methyl groups has been added (viscosity at 25°C, 400 cP) was used as a release agent of the packaging sheet.

### (Inventive Example 3)

A sample for use in the evaluation of peeling strength was prepared in the same manner as described in Inventive Example 1, except that a mixture prepared by adding 1 part by weight of bis(dodecylphenyl)iodonium hexafluoroantimonate to 100 parts by weight of an epoxy functional polydimethylsiloxane to which 3 mol % of 4-vinylcyclohexene oxide based on the total methyl group has been added (viscosity at 25°C, 400 cP) was used as a release agent of the packaging sheet.

### (Inventive Example 4)

A packaging sheet was prepared in the same manner as described in Inventive Example 1, except that a mixture prepared by adding 1 part by weight of bis(dodecylphenyl)iodonium hexafluoroantimonate to 100 parts by weight of an epoxy functional polydimethylsiloxane to which 10 mol % of 4-vinylcyclohexene oxide based on the total methyl groups has been added (viscosity at 25°C, 300 cP) was used as a release agent of the packaging sheet.

A hot melt type pressure-sensitive adhesive comprised of a styrene-isoprene hydrogenated copolymer was coated in a thickness of 45 µm on one side of a polypropylene/polyethylene blend film (weight ratio: 6/4, 95 µm in thickness) to prepare a pressure-sensitive adhesive tape (50 mm in width, 150 mm in length), and the release agent layer of the packaging sheet described above was laminated on the pressure-sensitive adhesive layer of the thus prepared pressure-sensitive adhesive tape, thereby preparing a sample for use in the evaluation of peeling strength.

### (Inventive Example 5)

A pressure-sensitive adhesive tape [trade name: No. 31B, manufactured by Nitto Denko Corporation] produced by coating an acrylic pressure-sensitive adhesive on one side of a polyester film was cut into a size of 50 mm in width and 150 mm in length, and the same packaging sheet of Inventive Example 1 was laminated on the pressure-sensitive adhesive layer of the tape to prepare a sample for use in the evaluation of peeling strength.

### (Inventive Example 6)

A sample for use in the evaluation of peeling strength was prepared by laminating the same packaging sheet of Inventive Example 2 on the pressure-sensitive adhesive layer of the same pressure-sensitive adhesive tape of Inventive Example 5.

### (Inventive Example 7)

A sample for use in the evaluation of peeling strength was prepared by laminating the same packaging sheet of Inventive Example 3 on the pressure-sensitive adhesive layer of the same pressure-sensitive adhesive tape of Inventive Example 5.

### (Inventive Example 8)

A sample for use in the evaluation of peeling strength was prepared by laminating the same packaging sheet of Inventive Example 4 on the pressure-sensitive adhesive layer of the same pressure-sensitive adhesive tape of Inventive Example 5.

### (Inventive Example 9)

A packaging sheet was prepared in the same manner as described in Inventive Example 1, except that the base material of the packaging sheet was changed to a polyethylene film of 30 µm in thickness.

A commercially available napkin having a pressure-sensitive adhesive (a napkin in which a pressure-sensitive adhesive layer is arranged on the main body for fixing it to underwear), from which its packaging material and the release paper protecting its pressure-sensitive adhesive layer have been removed, was packaged with the packaging sheet in such a manner that the release agent layer of the sheet contacted with the pressure-sensitive adhesive layer of the napkin, thereby preparing a packaged article containing a napkin.

### (Inventive Example 10)

A packaging sheet was prepared in the same manner as described in Inventive Example 2, except that the base material of the packaging sheet was changed to a polyethylene film of 30 µm in thickness.

The same napkin of Inventive Example 9 having a pressure-sensitive adhesive was packaged with the packaging sheet in such a manner that the release agent layer of the sheet contacted with the pressure-sensitive adhesive layer of the napkin, thereby preparing a packaged article containing a napkin.

### (Comparative Example 1)

A sample for use in the evaluation of peeling strength was prepared in the same manner as described in Inventive Example 1, except that a mixture prepared by adding 1 part by weight of bis(dodecylphenyl)iodonium hexafluoroantimonate to 100 parts by weight of an epoxy functional polydimethylsiloxane to which 30 mol % of 4-vinylcyclohexene oxide based on the total methyl groups has been added (viscosity at 25°C, 500 cP) was used as a release agent of the packaging sheet.

### (Comparative Example 2)

A release agent prepared by adding 2 parts by weight of an acetophenone based photoinitiator Dalocure 1173 (manufactured by Ciba-Geigy) to 100 parts by weight of a polyorganosiloxane RC-726/RC-711 mixture (7/3 in weight ratio) having acrylate groups (manufactured by Goldschmitt) was coated on one side of a polyethylene film having a thickness of 25 µm and subjected to ultraviolet ray irradiation under the following coating and irradiation conditions, thereby preparing a packaging sheet.

### Conditions for release agent coating

- Coating apparatus: offset gravure coater
- Coating amount: 1 g/m²
- Line speed: 20 m/min
- UV lamp: F-450 (H bulb) manufactured by FUSION
- Quantity of UV light: 120 W/cm
- The atmosphere for the ultraviolet ray irradiation was adjusted to an oxygen concentration of 50 ppm or less by nitrogen substitution.

A sample for use in the evaluation of peeling strength was prepared by laminating the release agent layer of the packaging sheet on the pressure-sensitive adhesive layer of the same pressure-sensitive adhesive tape of Inventive Example 1.

### (Comparative Example 3)

A polyorganosiloxane RC-726/RC-711 mixture (7/3 in weight ratio) having acrylate groups (manufactured by Goldschmitt) was coated on one side of a polyethylene film having a-thickness of 25 µm and subjected to electron ray irradiation under the following coating and irradiation conditions, thereby preparing a packaging sheet.

### Conditions for release agent coating

- Coating apparatus: offset gravure coater
- Coating amount: 1 g/m²
- Line speed: 20 m/min
- Electron ray irradiation apparatus: CB-150 ELECTROCURTAIN, manufactured by ESI
- Quantity of electron rays: 3 Mrad

A sample for use in the evaluation of peeling strength was prepared by laminating the release agent layer of the packaging sheet on the pressure-sensitive adhesive layer of the same pressure-sensitive adhesive tape of Inventive Example 1.

### (Comparative Example 4)

A sample for use in the evaluation of peeling strength was prepared by laminating the same packaging sheet of Comparative Example 1 on the pressure-sensitive adhesive layer of the same pressure-sensitive adhesive tape of Inventive Example 5.

### (Comparative Example 5)

A sample for use in the evaluation of peeling strength was prepared by laminating the same packaging sheet of Comparative Example 2 on the pressure-sensitive adhesive layer of the same pressure-sensitive adhesive tape of Inventive Example 5.

### (Comparative Example 6)

A sample for use in the evaluation of peeling strength was prepared by laminating the same packaging sheet of Comparative Example 3 on the pressure-sensitive adhesive layer of the same pressure-sensitive adhesive tape of Inventive Example 5.

### (Comparative Example 7)

A packaging sheet was prepared in the same manner as described in Comparative Example 1, except that the base material of the packaging sheet was changed to a polyethylene film of 30 µm in thickness.

The same napkin of Inventive Example 9 having a pressure-sensitive adhesive was packaged with the packaging sheet in such a manner that the release agent layer of the sheet contacted with the pressure-sensitive adhesive layer of the napkin, thereby preparing a packaged article containing a napkin.

### (Comparative Example 8)

A packaging sheet was prepared in the same manner as described in Comparative Example 2, except that the base material of the packaging sheet was changed to a polyethylene film of 30 µm in thickness.

The same napkin of Inventive Example 9 having a pressure-sensitive adhesive was packaged with the packaging sheet in such a manner that the release agent layer of the sheet contacted with the pressure-sensitive adhesive layer of the napkin, thereby preparing a packaged article containing a napkin.

### (Comparative Example 9)

A packaging sheet was prepared in the same manner as described in Comparative Example 3, except that the base material of the packaging sheet was changed to a polyethylene film of 30 µm in thickness.

The same napkin of Inventive Example 9 having a pressure-sensitive adhesive was packaged with the packaging sheet in such a manner that the release agent layer of the sheet contacted with the pressure-sensitive adhesive layer of the napkin, thereby preparing a packaged article containing a napkin.

### [Evaluation]

The samples for use in the evaluation of peeling strength obtained in Inventive Examples 1 to 8 and Comparative Examples 1 to 6 (cut into 25 mm in width) were allowed to stand under the following conditions to measure their peeling strength after the standing. In this case, the peeling strength was calculated by measuring, using a tensile tester, a force required to peel off the packaging sheet at an angle of 180° from the pressure-sensitive adhesive layer of each sample for use in the evaluation of peeling strength. The measurement by the tensile tester was carried out at a tensile tester speed of 300 mm/min.

### [Condition 1]

The samples for use in the evaluation of peeling strength were left outdoors for 3 days of fine weather. In this case, the samples were arranged with the packaging sheet as the front side, so that the packaging sheet side was directly exposed to sunlight.

### [Condition 2]

Ultraviolet rays were irradiated to the packaging sheet side of the samples for use in the evaluation of peeling strength, under the following conditions using a conveyor type ultraviolet ray irradiation apparatus.

### Ultraviolet ray irradiation conditions

- Light source: metal halide lamp (quantity of light, 80 W/cm)
- Conveyor speed: 5 m/min (irradiation distance, 145 mm)
- Frequency of irradiation: 10 times

### [Blank]

The samples for use in the evaluation of peeling strength were left in the dark at room temperature for 3 days.

Results of the measurement of peeling strength are shown in Table 1.

**Table 1**

| | | Peeling Strength (N/25 mm) | | |
|---|---|---|---|---|
| | | Condition 1 | Condition 2 | Blank |
| Inventive Example 1 | styrene-isoprene copolymer type | 0.26 (1.9) | 0.24 (1.7) | 0.14 |
| Inventive Example 2 | styrene-isoprene copolymer type | 0.19 (1.6) | 0.20 (1.7) | 0.12 |
| Inventive Example 3 | styrene-isoprene copolymer type | 0.17 (1.7) | 0.19 (1.9) | 0.10 |
| Inventive Example 4 | styrene-butadiene hydrogenated copolymer type | 0.34 (1.9) | 0.33 (1.8) | 0.18 |
| Inventive Example 5 | acrylic type | 0.07 (1.4) | 0.07 (1.4) | 0.05 |
| Inventive Example 6 | acrylic type | 0.05 (1.3) | 0.06 (1.5) | 0.04 |
| Inventive Example 7 | acrylic type | 0.04 (1.0) | 0.05 (1.3) | 0.04 |
| Inventive Example 8 | acrylic type | 0.09 (1.5) | 0.09 (1.5) | 0.06 |
| Comparative Example 1 | styrene-isoprene copolymer type | 3.22 (2.8) | 3.39 (3.0) | 1.14 |
| Comparative Example 2 | styrene-isoprene copolymer type | 5.01 (25) | 5.33 (27) | 0.20 |
| Comparative Example 3 | styrene-isoprene copolymer type | 1.66 (6.1) | 1.73 (6.4) | 0.27 |
| Comparative Example 4 | acrylic type | 0.98 (2.0) | 0.86 (1.8) | 0.48 |
| Comparative Example 5 | acrylic type | 0.40 (6.7) | 0.37 (6.2) | 0.06 |
| Comparative Example 6 | acrylic type | 0.12 (1.7) | 0.12 (1.7) | 0.07 |
| *Data in parentheses show increment of peeling strength (peeling strength of condition 1 or 2/peeling strength of blank). | | | | |

After allowing the packaged articles containing napkins obtained in Inventive Examples 9 and 10 and Comparative Examples 7 to 9 to stand under the same conditions as described above (condition 1, condition 2 and blank), peeling performance when the packaging sheet was peeled off from the napkin by hand was evaluated. In this case, the results were evaluated as "A" when peeling of the packaging sheet from the pressure-sensitive adhesive layer of napkin was easy, as "B" when it was slightly difficult or as "C" when it was considerably difficult.

Results of the evaluation of peeling performance are shown in Table 2.

**Table 2**

| | Peeling Strength (N/25 mm) | | |
|---|---|---|---|
| | Condition 1 | Condition 2 | Blank |
| Inventive Example 9 | A | A | A |
| Inventive Example 10 | A | A | A |
| Comparative Example 7 | C | C | A |
| Comparative Example 8 | C | C | A |
| Comparative Example 9 | B | B | A |
| A: The packaging sheet is easy to peel off. B: The packaging sheet is slightly difficult to peel off. C: The packaging sheet is considerably difficult to peel off. | | | |

In the packaging sheet of the present invention and the packaged articles containing a napkin having a pressure-sensitive adhesive, which are packaged with the packaging sheet, it is evident that the-tight release tendency (increment of peeling strength) is small when compared with Comparative Examples even if they are exposed to direct sunlight or left under an accelerating condition by force of ultraviolet ray irradiation.

Also, when the pressure-sensitive adhesive which constitutes the pressure-sensitive adhesive layer is a hot melt type pressure-sensitive adhesive comprised of a styrene-isoprene copolymer (an example of a type which contains aliphatic double bonds between carbon atoms in the molecule), it is evident that the tight release tendency (increment of peeling strength) becomes particularly small in comparison with Comparative Examples.

### INDUSTRIAL APPLICABILITY

Since the packaging sheet for sanitary napkin of the present invention has the aforementioned construction, even if the packaged article in which a napkin having a pressure-sensitive adhesive is individually packaged with such a packaging sheet, is left under an environment exposed to direct sunlight, increase in the peeling strength of the package sheet which protects the pressure-sensitive adhesive layer can be prevented.

Also, when the pressure-sensitive adhesive layer formed on the surface of napkins is composed of a pressure-sensitive adhesive having a polymer containing aliphatic double bonds between carbon atoms in the molecule as the main component, tight release tendency of the packaging sheet can be prevented particularly efficiently by combining the pressure-sensitive adhesive in combination with the packaging sheet of the present invention to prepare a packaged article containing a napkin.

In consequence, the problems involved in the prior art such as the difficulty in peeling off packaging sheet at the time of using a napkin can be solved by the use of the packaging sheet and the packaged article in which a sanitary napkin is packaged with the packaging sheet according to the present invention, so that excellent effects can be obtained for the stabilization of product qualities.

## Claims

1. A packaging sheet for a sanitary napkin in which a release agent layer is formed on one side of a base material, wherein the release agent layer is one prepared by curing a release agent containing an epoxy functional polyorganosiloxane as the main component by ultraviolet ray irradiation, the epoxy functional polyorganosiloxane containing a monovalent epoxy functional organic group in an amount of 1 to 20 mol % of the total organic groups.

2. The packaging sheet for a sanitary napkin according to claim 1 wherein said epoxy functional polyorganosiloxane which constitutes the release agent layer is comprised of a constituting unit represented by the following formula (1)
R¹ₘR²ₙSiO_{(4-m-n)/2} (1)
wherein R¹ is a hydrogen atom or a monovalent hydrocarbon group, R² is a hydrogen atom, a divalent hydrocarbon group, a monovalent hydrocarbon group or a monovalent epoxy functional organic group, and m and n each is an integer of from 0 to 3, with the proviso that the total of m and n is an integer of from 0 to 3.

3. A packaged article containing a sanitary napkin in which the napkin has a pressure-sensitive adhesive layer on its surface and is packaged in the packaging sheet of claim 1 or 2.

4. The packaged article containing a sanitary napkin according to claim 3, wherein the pressure-sensitive adhesive layer is comprised of a pressure-sensitive adhesive which contains a polymer having aliphatic double bonds between carbon atoms in the molecule as the main component.
